# EUROPÄISCHE PATENTSCHRIFT

(11) **EP 0 597 471 B1**
(45) Veröffentlichungstag und Bekanntmachung des Hinweises auf die Patenterteilung: **05.02.1997**
(21) Anmeldenummer: 93118277.8
(22) Anmeldetag: 11.11.1993
(51) Int. Cl.: A61B 17/34, A61B 17/00

(54) **Trokarhülse**
Trocar sleeve
Manchon de trocart

(30) Priorität: 12.11.1992 DE 4238144
(43) Veröffentlichungstag der Anmeldung: 18.05.1994
(73) Patentinhaber: Karl Storz GmbH & Co., D-78532 Tuttlingen (DE)
(72) Erfinder: Storz, Karl, Dr. med.h.c., D 78532 Tuttlingen (DE)
(74) Vertreter: Weller, Wolfgang, Dr.rer.nat.

(56) Entgegenhaltungen:
- EP-A- 0 400 458
- EP-A- 0 487 175
- WO-A-92/11882
- US-A- 5 108 408

## Beschreibung

Die Erfindung betrifft eine aus der DE-A-40 21 023 bekannte Trokarhülse zum Einführen eines Instrumentes, insbesondere einer Faßzange, in Körperhöhlen, insbesondere in den Douglas-Raum, mit einem Klappventil.

Aus der DE-A-40 21 023 ist ein Trokar mit einer Trokarhülse bekannt, die durch einen Trokardorn verschlossen werden kann. Der Trokardorn weist eine kegelige oder dreikantige Spitze zum Durchstechen von Gewebe, insbesondere der Bauchdecke, auf.

Aus dem Dokument EP-A-0 487 175 ist ein Trokar ersichtlich, bei dem am patientennahen Ende ein aufblasbarer Ballon vorgesehen ist, der nach Einbringen der Trokarhülse in den Körper aufgeblasen wird. Dieser aufgeblasene Ballon dient als Gegendruckstück zu einem weiteren Anlagestück, das von der Außenseite an den Körper angelegt wird, so daß dann zwischen dem Ballon und dem angelegten Gegenstück die Bauchdecke gehalten ist.

Aus dem Dokument EP-A-0 400 458 ist eine Kanüle für die Laparoskopie ersichtlich, die ein kegelstumpfartiges Bauelement aufweist, deren Spitze sich aber am patientennahen Ende in einem festen, zunächst in der Kanülenachse fortstreckenden, anschließend seitlich abgewinkelten Stück fortsetzt.

Aus dem Dokument US-A-5 108 408 ist ein medizinisches Instrument ersichtlich, das am patientennahen Ende mit einem aufstellbaren Ring versehen ist, der zum Einführen in bzw. zum Abziehen von Körperhöhlen flachgestellt wird.

Soll mit einem derartigen Trokar eine Wand im Douglas-Raum durchstochen werden, besteht die Gefahr, daß der Trokardorn den Darm beschädigt.

Aus dem DE-GM 71 45 806 ist ferner ein Trokar bekannt, der von einem Mantelrohr umgeben ist, das mit einer Trokarhülse einen Luftkanal bildet und Öffnungen für den Luftdurchgang aufweist. Derartige Trokare werden bei Operationen in der Bauchhöhle oder in der Brusthöhle verwendet. Dabei wird dann unter anderem eine Insuflations-Hülse mit einem stumpfen Ende anstelle des scharfen Dornes in die Trokarhülse eingeführt. Beispielsweise kann hier in der Trokarhülse eine Einsatzhülse angeordnet sein, in der zwei Operationsinstrumente angeordnet sind. Dabei kann die Einsatzhülse eine Membrandichtung mit zwei im Abstand zueinander angeordneten Bohrungen für Operationsinstrumente aufweisen.

Bei einem weiteren bekannten Trokar besteht die Möglichkeit, die Verbindung des Mantelrohres mit der Trokarhülse leicht zu lösen, so daß der Luftkanal leicht gereinigt werden kann.

Aufgabe der vorliegenden Erfindung ist, eine Trokarhülse der eingangs genannten Art derart zu verbessern, daß eine Gewebewand, insbesondere die transvaginale Douglas-Wand, durchtrennt werden kann, ohne daß die Gefahr der Beschädigung von weiteren Körperteilen, beispielsweise des Darmes, besteht.

Erfindungsgemäß wird die Aufgabe dadurch gelöst, daß die Trokarhülse am patientennahen Ende ein balliges Element aufweist, das mit einem Schlitz versehen ist, der als Führung für ein Inzisionsinstrument dient.

Aufgrund des balligen Elements kann die Trokarhülse in Körperöffnungen oder Körperhöhlen, wie beispielsweise das hintere Scheidengewölbe, einfach eingeführt werden. Einfach eingeführt werden bedeutet dabei, daß aufgrund der balligen, somit abgerundeten Form das patientennahe Ende der Trokarhülse durch Körperöffnungen, beispielweise durch die Vagina, gegebenenfalls unter Aufspreizen hindurchbewegt werden und an eine Wand im Körper angelegt werden kann. Dies ist von der handhabenden Person einfach zu bewerkstelligen, und ist ohne Beeinträchtigung des Körpers durchzuführen. Das ballige Element weist dabei einen wesentlich größeren Außendurchmesser auf als der verbleibende Teil der Trokarhülse. Demzufolge liegt die Trokarhülse im Bereich des balligen Elements über einen relativ großen Flächenbereich an der entsprechenden Wand, beispielsweise der transvaginalen Douglas-Wand, an. Es ist nun möglich, von der anderen Seite der Wand her eine Inzision durchzuführen, wobei der Schlitz am balligen Element als Führung für ein Inzisionsinstrument dient. Als Inzisionsinstrument kann beispielsweise ein mechanisches Schneideinstrument, ein Laserschneideelement oder eine Nadelelektrode eingesetzt werden. Dadurch, daß das ballige Element flächig an der Wand anliegt, an der die Inzision durchgeführt werden soll und daß ferner der Führungsschlitz vorgesehen ist, kann die Inzision an einer exakt vorbestimmten Stelle und kontrolliert durchgeführt werden, ohne daß die Gefahr besteht, daß versehentlich andere Körperteile, beispielsweise ein Darm, verletzt bzw. durchstochen werden, wie das bei dem Durchstechen von derartigen Wänden mit einem Trokardorn möglich ist. Nach Anbringen der Inzision im Bereich der Mündung der Trokarhülse kann durch diese ein beliebiges Instrument, beispielsweise eine Faßzange, hindurchgeschoben werden, um abgetrennte Gewebeteile zu erfassen. Beispielsweise können von einer durch die Trokarhülse durchgeschobene Faßzange laparoskopisch abgetrennte Gewebeteile im Douglas-Raum erfaßt und, falls diese relativ klein sind, durch die Trokarhülse hindurch entfernt werden. Sind die von der Faßzange ergriffenen Gewebeteile relativ groß, so kann die Trokarhülse samt der darin aufgenommenen Faßzange sowie den von dieser ergriffenen Gewebeteilen vom Körper abgezogen werden. Beim Abziehen der Trokarhülse, beispielsweise aus der Vagina, weitet das ballige Element die entsprechende Körperhöhle auf, so daß die am nachlaufenden Ende überstehende Faßzange samt dem davon ergriffenen Gewebe einfach abgezogen werden kann, ohne daß die erfaßten Gewebeteile verloren werden. Das von dem balligen Element gespreizte Gewebe schließt sich am nachlaufenden Ende des balligen Elements, von dem Teile der Faßzange mit dem ergriffenen Gewebe vorstehen, erst mit einer solchen Zeitverzögerung, daß die Gefahr herabgesetzt ist, daß von der Faßzange ergriffene Gewebeteile zurückbehalten werden. Strömungstechnisch ausgedrückt kann man das so erläutern, daß die über das nachlaufende Ende des balligen Elements vorstehenden Teile von Faßzange und erfaßtem Gewebe quasi im "Windschatten" des sich bewegenden balligen Elements befinden. Um eine Dissemination zu verhindern, kann auch das von der Faßzange erfaßte Gewebe vor der Extraktion in einen Extraktionsbeutel eingeschoben werden.

In einer besonderen Ausgestaltung der Erfindung ist das ballige Element als Kugel ausgebildet.

Diese Maßnahme hat nun den Vorteil, daß aufgrund der Kugelgeometrie sowohl beim Einführen als auch beim Abziehen der Trokarhülse das entsprechend damit in Berührung stehende Gewebe sanft seitlich bewegt werden kann.

Weitere vorteilhafte Ausgestaltungen sind den anderen Unteransprüchen zu entnehmen.

Weitere Vorteile und Einzelheiten der Erfindung ergeben sich aus der folgenden Beschreibung eines Ausführungsbeispiels im Zusammenhang mit den Zeichnungen.

Es zeigen:
- Fig. 1: eine perspektivische Ansicht eines Ausführungsbeispiels einer erfindungsgemäßen Trokarhülse,
- Fig. 2: schematisch eine Seitenansicht eines bestimmten Operationsvorganges unter Verwendung einer erfindungsgemäßen Trokarhülse, und
- Fig. 3: eine der Schnittdarstellung von Fig. 2 entsprechende Darstellung eines zeitlich späteren Abschnittes eines Operationsvorganges.

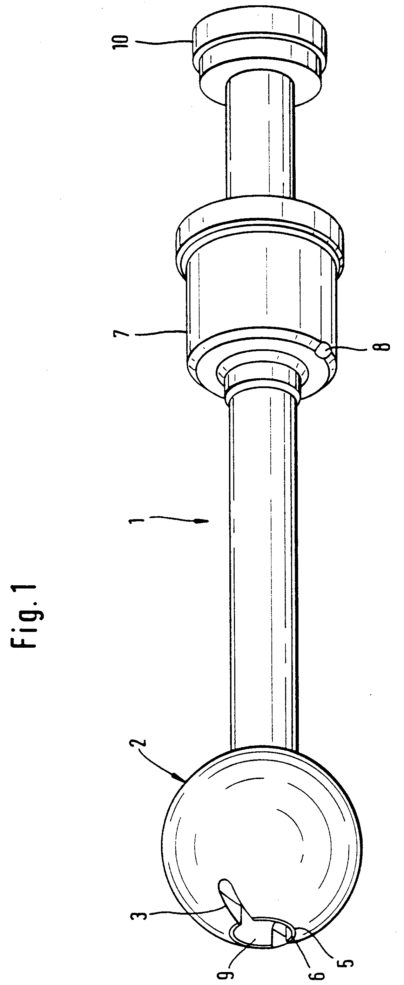

In Fig. 1 ist eine Trokarhülse 1 aus Metall dargestellt, die ein Klappventil 7 aufweist, das mit einem hier nicht näher bezeichneten Schraubverschluß versehen ist. Trokarhülsen mit Klappventilen sind dem Fachmann bestens bekannt, und müssen daher nicht näher im einzelnen dargestellt und beschrieben werden. Das Klappventil 7 ist mit einer Markierung 8 vorgesehen, deren Sinn und Zweck nachfolgend noch erläutert wird.

Am patientennahen Ende ist die Trokarhülse 1 mit einem balligen Element in Form einer Kugel 2 versehen, durch die mittig hindurch die Trokarhülse 1 reicht. Die Kugel 2 ist aus einem isolierenden Material hergestellt und kann, je nach Einsatzgebiet, verschiedene Außendurchmesser aufweisen. Typische Außendurchmesser sind beispielsweise 35 mm oder 40 mm.

Die Kugel 2 ist mit einer inneren durchgehenden Öffnung versehen, deren Innendurchmesser 5 so bemaßt ist, daß die Trokarhülse 1 in dieser Öffnung im Paßsitz aufgenommen ist. Der Innendurchmesser 6 der Trokarhülse 1 beträgt beispielsweise 10 mm.

Es ist selbstverständlich auch möglich, die Trokarhülse 1 außen an der Kugel 2 anzusetzen und dieser lediglich eine entsprechende durchgehende Bohrung zu verleihen.

Im Bereich der Mündung der durch die Kugel 2 durchgehenden Öffnung 9 am patientennahen Ende ist ein Schlitz 3 vorgesehen, der im dargestellten Ausführungsbeispiel eine seitliche Erweiterung der Öffnung 9 darstellt.

Der Schlitz 3, der beispielsweise als eine eingeschnittene Nut ausgebildet sein kann, steht in einer bestimmten Ausrichtung zu der zuvor erwähnten Markierung 8 an dem Gehäuses des Klappventils 7.

Wie nachfolgend noch anhand der Handhabung der erfindungsgemäßen Trokarhülse 1 beschrieben wird, dient der Schlitz 3 als Führung und Orientierungshilfe beim Anbringen einer Inzision im Bereich des Austrittes der Öffnung 9 am patientennahen Ende der Kugel 2, wenn diese an einer Gewebewand liegt.

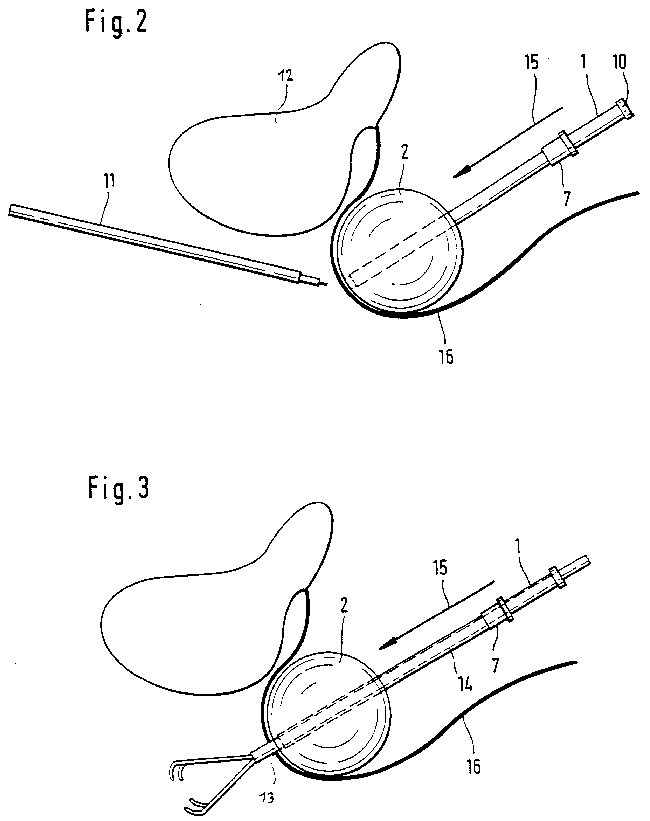

In Fig. 2 ist eine Situation dargestellt, bei der die in Fig. 1 näher beschriebene Trokarhülse 1 über eine Vagina in das hintere Scheidengewölbe eingeführt wurde, wie das durch einen Pfeil 15 angedeutet ist. Benachbart zum Scheidengewölbe liegt die Gebärmutter 12.

In der in Fig. 2 dargestellten Position kommt die Kugel 2 an der Wand 16 des Douglas-Raumes zum Liegen. Die korrekte Plazierung kann visuell durch ein Laparoskop überprüft werden. Nachdem die Kugel 2 korrekt an der Wand 16 angelegt wurde, kann über eine Nadelelektrode 11 endoskopisch in Höhe des Douglas-Raumes eine Inzision angebracht werden. Der Schlitz 3 sowie die übrige Öffnung 9 am patientennahen Ende der Kugel 2 dienen dabei als eine Art Schablone bzw. Führung zum Durchtrennen der Wand 16 mittels der Nadelelektrode 11. Die Markierung 8 am Klappventilgehäuse dient als Ausrichtungshilfe. Die Inzision wird somit von der Seite der Wand 16 her durchgeführt, die gegenüberliegend zu der Seite ist, an der die Kugel 2 angelegt ist. Durch diese Art und Weise kann die Gefahr der Beschädigung eines Darmes stark verringert, wenn nicht gar völlig beseitigt werden. Nach Anbringen des Schnittes durch eine suprapubische abdominelle Inzision ist ein CO₂-Verlust durch die Kuldotomie nicht zu befürchten, solange die Kugel 2 an dem Scheidengewölbe angelegt bleibt.

In Fig. 3 ist ersichtlich, daß zwischenzeitlich durch die Trokarhülse 1 und die Öffnung 13 in der Wand 16, die zuvor durch die Nadelelektrode 11 erzeugt wurde, eine Faßzange 14 hindurchgeschoben wurde. Es ist zu erkennen, daß die Faßzange 14 an ihrem patientennahen Ende schon gespreizt ist, um beispielsweise ein laparoskopisch abgetrenntes Gewebeteil über den Douglas-Raum zu erfassen. Das zu extrahierende Gewebe kann unter endoskopischer Kontrolle mit der Zange 14 erfaßt werden. Bei Verdacht auf Malignität und Dermoidzysten wird das abgetrennte Gewebe vor der Extraktion in einen Extraktionsbeutel eingeführt, um eine parietale Kontamination zu vermeiden.

Anschließend wird die Faßzange 14 soweit zurückgezogen, bis das Gewebe die Kugel 2 berührt, wobei die Faßzange vollständig geschlossen ist und das erfaßte Gewebeteil fest hält. Danach wird die Trokarhülse 1 samt Faßzange 14 und dem von dieser erfaßten Gewebe entfernt, so daß das im Douglas-Raum abgetrennte Gewebe durch die Öffnung 13 in der Wand 14 über die Vagina entfernt werden kann. Diese Vorgehensweise erlaubt die Extraktion von Gewebe bis zu einer Größe von 6 bis 7 cm.

Nach Entfernen des Gewebes kann die Trokarhülse 1 ohne Faßzange 14 unter endoskopischer Kontrolle erneut in das Scheidengewölbe eingeführt werden, um das Entweichen von Gas während der restlichen Operationszeit zu vermeiden.

Im konkreten Ausführungsbeispiel wurde das ballige Element in Form einer Kugel 2 beschrieben, es ist selbstverständlich möglich, andere, elliptische, eiförmige, eben ballige und abgerundete Geometrien einzusetzen, um denselben Erfolg zu erzielen.

## Patentansprüche

1. Trokarhülse zum Einführen eines Instrumentes, insbesondere einer Faßzange (14), in Körperhöhlen, insbesondere in den Douglas-Raum, mit einem Klappventil (7), dadurch gekennzeichnet, daß am patientennahen Ende die Trokarhülse (1) ein balliges Element (2) aufweist, das mit einem Schlitz (3) versehen ist, der als Führung für ein Inzisionsinstrument dient.

2. Trokarhülse nach Anspruch 1, dadurch gekennzeichnet, daß das ballige Element (2) jeweils eine solche Größe aufweist, daß die Trokarhülse (1) durch Körperöffnungen unter Aufweitung derselben, insbesondere eine Vagina, bewegbar ist.

3. Trokarhülse nach Anspruch 1 oder 2, dadurch gekennzeichnet, daß das ballige Element (2) aus einem isolierten Körper besteht.

4. Trokarhülse nach einem der Ansprüche 1 bis 3, dadurch gekennzeichnet, daß das ballige Element (2) eine durchgehende Öffnung (9) in Verlängerung des Trokar-Kanals (10) aufweist, und daß der Schlitz (3) als seitliche Erweiterung dieser Öffnung (9) ausgebildet ist.

5. Trokarhülse nach einem der Ansprüche 1 bis 4, dadurch gekennzeichnet, daß das ballige Element als Kugel (2) ausgebildet ist.

6. Trokarhülse nach einem der Ansprüche 1 bis 5, dadurch gekennzeichnet, daß an einem Gehäuse des Klappventiles (7) eine Markierung (8) zur Positionierung des Schlitzes (3) angeordnet ist.

## Claims

1. Trocar tube for introducing an instrument, in particular forceps (14), into body cavities, in particular into the Douglas's pouch, with a flap valve (7), characterised in that the trocar tube (1) has, at the end close to the patient, a ball-shaped element (2) provided with a slot (3) acting as a guide for an incision instrument.

2. Trocar tube according to claim 1, characterised in that the ball-shaped element (2) is of such a size in each case that the trocar tube (1) can be moved through body orifices while enlarging them, in particular a vagina.

3. Trocar tube according to claim 1 or 2, characterised in that the ball-shaped element (2) consists of an insulated body.

4. Trocar tube according to one of claims 1 to 3, characterised in that the ball-shaped element (2) has a continuous orifice (9) extending the trocar duct (10) and in that the slot (3) is designed as a lateral enlargement of this orifices (9).

5. Trocar tube according to one of claims 1 to 4, characterised in that the ball-shaped element is designed as a sphere (2).

6. Trocar tube according to one of claims 1 to 5, characterised in that a marking (8) for positioning the slot (3) is arranged on a housing of the flap valve (7).

## Revendications

1. Douille de trocart pour introduire un instrument, notamment une pince de saisie (14) dans des cavités du corps, notamment dans le cul-de-sac de Douglas, avec une vanne de fermeture (7), caractérisée en ce que la douille de trocart (1) présente à l'extrémité proche du patient un élément bombé (2) qui est muni d'une fente (3), laquelle sert de guidage pour un instrument d'incision.

2. Douille de trocart selon la revendication 1, caractérisée en ce que l'élément bombé (2) présente une taille telle que la douille de trocart (1) peut être déplacée à travers des ouvertures corporelles, notamment un vagin, avec effet d'élargissement de celles-ci.

3. Douille de trocart selon la revendication 1 ou 2, caractérisée en ce que l'élément bombé (2) est constitué d'un corps isolé.

4. Douille de trocart selon l'une des revendications 1 à 3, caractérisée en ce que l'élément bombé (2) présente une ouverture continue (9) dans le prolongement du canal de trocart (10), et en ce que la fente (3) est conformée en élargissement latéral de cette ouverture (9).

5. Douille de trocart selon l'une des revendications 1 à 4, caractérisée en ce que l'élément bombé est conformé en sphère (2).

6. Douille de trocart selon l'une des revendications 1 à 5, caractérisée en ce qu'un marquage (8) est placé sur le boîtier de la vanne de fermeture (7) pour le positionnement de la fente (3).
